# EUROPEAN PATENT APPLICATION

(11) **EP 2 746 292 A1**
(43) Date of publication of application: **25.06.2014**
(21) Application number: 13198692.9
(22) Date of filing: 20.12.2013
(51) Int. Cl.: C07K 16/24, A61P 13/12

(54) **Anti-IL21 antibodies for use in the treatment of nephropathies**

(30) Priority: 21.12.2012 EP 12198934; 16.01.2013 US 201361753129 P
(71) Applicant: Novo Nordisk A/S, 2880 Bagsværd (DK)
(72) Inventor: Rosendahl, Alexander, 21853 Klagshamn (SE); Mayer, Christopher, 2920 Charlottenlund (DK); Cucak, Helena, 211 49 Malmö (SE); Nielsen Fink, Lisbeth, 2100 Copenhagen O (DK)

(57) **Abstract**

The present invention relates to treatment of nephropathy. In particular, the present invention relates to treatment of nephropathy using therapeutic antibodies.

SEQUENCE LISTING

<110>  Novo Nordisk A/S

<120>  Treatment of Nephropathy

<130>  8614

<160>  7

<170>  PatentIn version 3.5

<210>  1
<211>  162
<212>  PRT
<213>  homo sapiens

<400>  1

Met Arg Ser Ser Pro Gly Asn Met Glu Arg Ile Val Ile Cys Leu Met
1               5                   10                  15

Val Ile Phe Leu Gly Thr Leu Val His Lys Ser Ser Ser Gln Gly Gln
            20                  25                  30

Asp Arg His Met Ile Arg Met Arg Gln Leu Ile Asp Ile Val Asp Gln
        35                  40                  45

Leu Lys Asn Tyr Val Asn Asp Leu Val Pro Glu Phe Leu Pro Ala Pro
    50                  55                  60

Glu Asp Val Glu Thr Asn Cys Glu Trp Ser Ala Phe Ser Cys Phe Gln
65                  70                  75                  80

Lys Ala Gln Leu Lys Ser Ala Asn Thr Gly Asn Asn Glu Arg Ile Ile
                85                  90                  95

Asn Val Ser Ile Lys Lys Leu Lys Arg Lys Pro Pro Ser Thr Asn Ala
            100                 105                 110

Gly Arg Arg Gln Lys His Arg Leu Thr Cys Pro Ser Cys Asp Ser Tyr
        115                 120                 125

Glu Lys Lys Pro Pro Lys Glu Phe Leu Glu Arg Phe Lys Ser Leu Leu
    130                 135                 140

Gln Lys Met Ile His Gln His Leu Ser Ser Arg Thr His Gly Ser Glu
145                 150                 155                 160

Asp Ser

<210>  2
<211>  213
<212>  PRT
<213>  artificial

<220>
<223>  mAb 5 light chain

<400>  2

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Trp Thr
                85                  90                  95

Phe Gly Gln Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys

    210

<210>  3
<211>  456
<212>  PRT
<213>  artificial

<220>
<223>  mAb 5 heavy chain

<400>  3

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Phe Ile Trp Tyr Asp Gly Ser Asp Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Gly Asp Ser Ser Asp Trp Tyr Gly Asp Tyr Tyr Phe Gly
            100                 105                 110

Met Asp Val Trp Gly Gln Gly Thr Thr Val Thr Val Ser Ser Ala Ser
        115                 120                 125

Thr Lys Gly Pro Ser Val Phe Pro Leu Ala Pro Ser Ser Lys Ser Thr
    130                 135                 140

Ser Gly Gly Thr Ala Ala Leu Gly Cys Leu Val Lys Asp Tyr Phe Pro
145                 150                 155                 160

Glu Pro Val Thr Val Ser Trp Asn Ser Gly Ala Leu Thr Ser Gly Val
                165                 170                 175

His Thr Phe Pro Ala Val Leu Gln Ser Ser Gly Leu Tyr Ser Leu Ser
            180                 185                 190

Ser Val Val Thr Val Pro Ser Ser Ser Leu Gly Thr Gln Thr Tyr Ile
        195                 200                 205

Cys Asn Val Asn His Lys Pro Ser Asn Thr Lys Val Asp Lys Lys Val
    210                 215                 220

Glu Pro Lys Ser Cys Asp Lys Thr His Thr Cys Pro Pro Cys Pro Ala
225                 230                 235                 240

Pro Glu Ala Glu Gly Ala Pro Ser Val Phe Leu Phe Pro Pro Lys Pro
                245                 250                 255

Lys Asp Thr Leu Met Ile Ser Arg Thr Pro Glu Val Thr Cys Val Val
            260                 265                 270

Val Asp Val Ser His Glu Asp Pro Glu Val Lys Phe Asn Trp Tyr Val
        275                 280                 285

Asp Gly Val Glu Val His Asn Ala Lys Thr Lys Pro Arg Glu Glu Gln
    290                 295                 300

Tyr Asn Ser Thr Tyr Arg Val Val Ser Val Leu Thr Val Leu His Gln
305                 310                 315                 320

Asp Trp Leu Asn Gly Lys Glu Tyr Lys Cys Lys Val Ser Asn Lys Ala
                325                 330                 335

Leu Pro Ser Ser Ile Glu Lys Thr Ile Ser Lys Ala Lys Gly Gln Pro
            340                 345                 350

Arg Glu Pro Gln Val Tyr Thr Leu Pro Pro Ser Arg Asp Glu Leu Thr
        355                 360                 365

Lys Asn Gln Val Ser Leu Thr Cys Leu Val Lys Gly Phe Tyr Pro Ser
    370                 375                 380

Asp Ile Ala Val Glu Trp Glu Ser Asn Gly Gln Pro Glu Asn Asn Tyr
385                 390                 395                 400

Lys Thr Thr Pro Pro Val Leu Asp Ser Asp Gly Ser Phe Phe Leu Tyr
                405                 410                 415

Ser Lys Leu Thr Val Asp Lys Ser Arg Trp Gln Gln Gly Asn Val Phe
            420                 425                 430

Ser Cys Ser Val Met His Glu Ala Leu His Asn His Tyr Thr Gln Lys
        435                 440                 445

Ser Leu Ser Leu Ser Pro Gly Lys

450                 455

<210>  4
<211>  214
<212>  PRT
<213>  artificial

<220>
<223>  mAb 14 light chain

<400>  4

Ala Ile Gln Leu Thr Gln Ser Pro Ser Ser Leu Ser Ala Ser Val Gly
1               5                   10                  15

Asp Arg Val Thr Ile Thr Cys Arg Ala Ser Gln Asp Ile Asp Ser Ala
            20                  25                  30

Leu Ala Trp Tyr Gln Gln Lys Pro Gly Lys Ala Pro Lys Ile Leu Ile
        35                  40                  45

His Asp Ala Ser Ser Leu Glu Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Phe Asn Ser Tyr Pro Tyr
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  5
<211>  447
<212>  PRT
<213>  artificial

<220>
<223>  mAb 14 heavy chain

<400>  5

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Ile Phe Ser Ser Tyr
            20                  25                  30

Ser Met Asn Trp Val Arg Gln Ala Pro Gly Lys Gly Leu Glu Trp Val
        35                  40                  45

Ser Ser Ile Thr Ser Gly Ser Tyr Tyr Ile His Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Val Arg Glu Arg Gly Trp Gly Tyr Tyr Gly Met Asp Val Trp Gly Gln
            100                 105                 110

Gly Thr Thr Val Thr Val Ser Ser Ala Ser Thr Lys Gly Pro Ser Val
        115                 120                 125

Phe Pro Leu Ala Pro Cys Ser Arg Ser Thr Ser Glu Ser Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Lys Thr Tyr Thr Cys Asn Val Asp His Lys
        195                 200                 205

Pro Ser Asn Thr Lys Val Asp Lys Arg Val Glu Ser Lys Tyr Gly Pro
    210                 215                 220

Pro Cys Pro Ser Cys Pro Ala Pro Glu Phe Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gly Leu Pro Ser Ser Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Arg Leu Thr Val Asp Lys Ser Arg
                405                 410                 415

Trp Gln Glu Gly Asn Val Phe Ser Cys Ser Val Met His Glu Ala Leu
            420                 425                 430

His Asn His Tyr Thr Gln Lys Ser Leu Ser Leu Ser Leu Gly Lys
        435                 440                 445

<210>  6
<211>  538
<212>  PRT
<213>  homo sapiens

<400>  6

Met Pro Arg Gly Trp Ala Ala Pro Leu Leu Leu Leu Leu Leu Gln Gly
1               5                   10                  15

Gly Trp Gly Cys Pro Asp Leu Val Cys Tyr Thr Asp Tyr Leu Gln Thr
            20                  25                  30

Val Ile Cys Ile Leu Glu Met Trp Asn Leu His Pro Ser Thr Leu Thr
        35                  40                  45

Leu Thr Trp Gln Asp Gln Tyr Glu Glu Leu Lys Asp Glu Ala Thr Ser
    50                  55                  60

Cys Ser Leu His Arg Ser Ala His Asn Ala Thr His Ala Thr Tyr Thr
65                  70                  75                  80

Cys His Met Asp Val Phe His Phe Met Ala Asp Asp Ile Phe Ser Val
                85                  90                  95

Asn Ile Thr Asp Gln Ser Gly Asn Tyr Ser Gln Glu Cys Gly Ser Phe
            100                 105                 110

Leu Leu Ala Glu Ser Ile Lys Pro Ala Pro Pro Phe Asn Val Thr Val
        115                 120                 125

Thr Phe Ser Gly Gln Tyr Asn Ile Ser Trp Arg Ser Asp Tyr Glu Asp
    130                 135                 140

Pro Ala Phe Tyr Met Leu Lys Gly Lys Leu Gln Tyr Glu Leu Gln Tyr
145                 150                 155                 160

Arg Asn Arg Gly Asp Pro Trp Ala Val Ser Pro Arg Arg Lys Leu Ile
                165                 170                 175

Ser Val Asp Ser Arg Ser Val Ser Leu Leu Pro Leu Glu Phe Arg Lys
            180                 185                 190

Asp Ser Ser Tyr Glu Leu Gln Val Arg Ala Gly Pro Met Pro Gly Ser
        195                 200                 205

Ser Tyr Gln Gly Thr Trp Ser Glu Trp Ser Asp Pro Val Ile Phe Gln
    210                 215                 220

Thr Gln Ser Glu Glu Leu Lys Glu Gly Trp Asn Pro His Leu Leu Leu
225                 230                 235                 240

Leu Leu Leu Leu Val Ile Val Phe Ile Pro Ala Phe Trp Ser Leu Lys
                245                 250                 255

Thr His Pro Leu Trp Arg Leu Trp Lys Lys Ile Trp Ala Val Pro Ser
            260                 265                 270

Pro Glu Arg Phe Phe Met Pro Leu Tyr Lys Gly Cys Ser Gly Asp Phe
        275                 280                 285

Lys Lys Trp Val Gly Ala Pro Phe Thr Gly Ser Ser Leu Glu Leu Gly
    290                 295                 300

Pro Trp Ser Pro Glu Val Pro Ser Thr Leu Glu Val Tyr Ser Cys His
305                 310                 315                 320

Pro Pro Arg Ser Pro Ala Lys Arg Leu Gln Leu Thr Glu Leu Gln Glu
                325                 330                 335

Pro Ala Glu Leu Val Glu Ser Asp Gly Val Pro Lys Pro Ser Phe Trp
            340                 345                 350

Pro Thr Ala Gln Asn Ser Gly Gly Ser Ala Tyr Ser Glu Glu Arg Asp
        355                 360                 365

Arg Pro Tyr Gly Leu Val Ser Ile Asp Thr Val Thr Val Leu Asp Ala
    370                 375                 380

Glu Gly Pro Cys Thr Trp Pro Cys Ser Cys Glu Asp Asp Gly Tyr Pro
385                 390                 395                 400

Ala Leu Asp Leu Asp Ala Gly Leu Glu Pro Ser Pro Gly Leu Glu Asp
                405                 410                 415

Pro Leu Leu Asp Ala Gly Thr Thr Val Leu Ser Cys Gly Cys Val Ser
            420                 425                 430

Ala Gly Ser Pro Gly Leu Gly Gly Pro Leu Gly Ser Leu Leu Asp Arg
        435                 440                 445

Leu Lys Pro Pro Leu Ala Asp Gly Glu Asp Trp Ala Gly Gly Leu Pro
    450                 455                 460

Trp Gly Gly Arg Ser Pro Gly Gly Val Ser Glu Ser Glu Ala Gly Ser

465                 470                 475                 480

Pro Leu Ala Gly Leu Asp Met Asp Thr Phe Asp Ser Gly Phe Val Gly
                485                 490                 495

Ser Asp Cys Ser Ser Pro Val Glu Cys Asp Phe Thr Ser Pro Gly Asp
            500                 505                 510

Glu Gly Pro Pro Arg Ser Tyr Leu Arg Gln Trp Val Val Ile Pro Pro
        515                 520                 525

Pro Leu Ser Ser Pro Gly Pro Gln Ala Ser
    530                 535

<210>  7
<211>  369
<212>  PRT
<213>  homo sapiens

<400>  7

## Description

### TECHNICAL FIELD

The present invention relates to treatment of nephropathy. In particular, the present invention relates to use of therapeutic antibodies for treating inflammatory nephropathy.

### BACKGROUND

Nephropathy (Kidney Disease) means damage to or disease of a kidney. "Nephrosis" is a non-inflammatory kidney disease. "Nephritis" is an inflammatory kidney disease. Nephropathy is a chronic non-communicable disease, having serious consequence if it cannot be controlled effectively. In general, there is a need in the art for alternative and preferably improved treatment options.

The symptoms of e.g. diabetic nephropathy are currently treated with ACE inhibitor drugs which reduce proteinuria levels and slow disease progression. The renal protection effect is related to the antihypertensive effects in normal and hypertensive patients. There is thus a great need in the art for alternative and/or improved treatment options of this and other serious chronic conditions related to kidney malfunction.

### SUMMARY

The present invention relates to use of IL-21 antibodies, wherein said antibodies compete with a receptor for binding IL-21, for treatment of nephropathy, preferably inflammatory nephropathy (nephritis). Such drugs have the potential to provide alternative and preferably improved treatment options for kidney patients.

### DESCRIPTION

The pathology of diabetic nephropathy includes apoptosis of glomeruli cells and/or fibrosis in tubular cells. The data shown herein, show that media from Th17 cultures, which are dependent on IL-21, induces apoptosis of glomeruli cells. Moreover, proximal tubular epithelial cells (PTECs) acquire IL-21Rα expression during disease progression, suggesting that IL-21 signalling is involved in the fibrotic responses in this and other inflammatory kidney diseases.

### BRIEF DESCRIPTION OF DRAWINGS

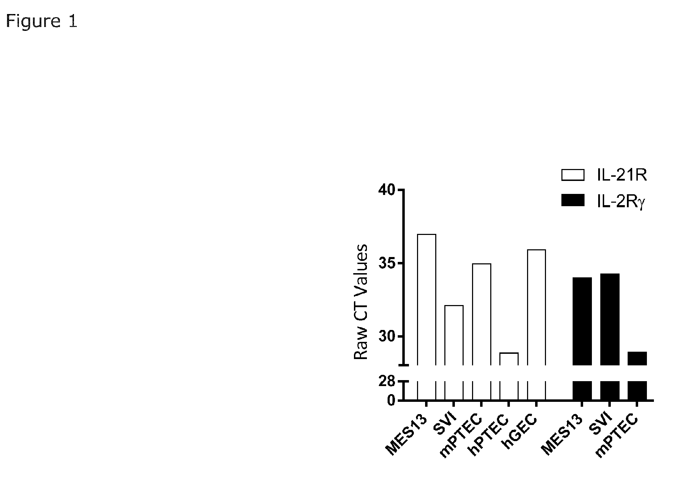

**Figure 1****:** Expression of IL21 R and IL2γ in mouse and human kidney cells. Total RNA was isolated from MES13 mouse mesangial cells, SVI40 mouse podocyte cells, mPTEC mouse primary proximal tubular epithelial cells, hPTEC human primary proximal tubular epithelial cells and hGEC human glomerular endothelial cells using Rneasy kit (Qiagen). qPCR was run on the samples using Taqman primers/probes for IL21 R and IL2γ. Data is shown as raw CT (cycle threshold) values, where lower CT values equal higher expression.

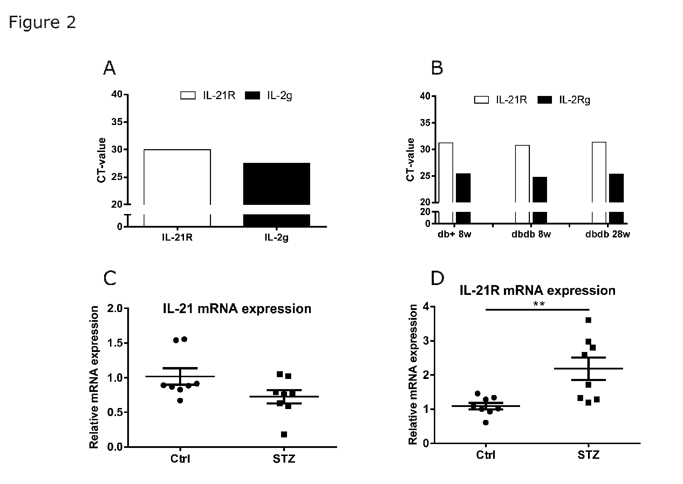

**Figure 2****:** Expression of IL21, IL21 R and IL2γ in mouse kidney cells. Total RNA was isolated and qPCR was run on the samples using Taqman primers/probes for IL21R and IL2γ. A. Nondiabetic C57B6J mouse kidney. B. 8 week old db+ and dbdb and 28 week old dbdb mouse glomeruli. Data is shown as CT values. C, D. Control and streptozotocin treated mouse whole kidney. Data is shown as relative mRNA expression, normalized to the average of two housekeeping genes, Rps13 and RpI27, with SEM for 8 animals. ** p<0.01.

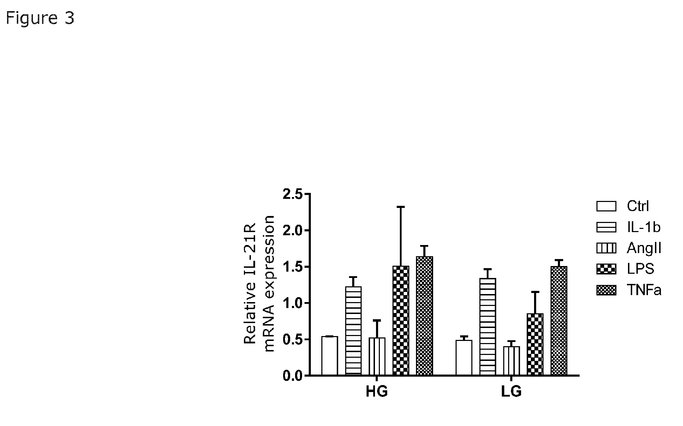

**Figure 3****:** IL1 beta, LPS and TNFalpha induce IL21 R mRNA expression in human GECs. Human glomerular endothelial cells were treated with IL-1 beta (10 ng/ml), angiotensin II (1 uM), LPS (100 ng/ml) and TNFalpha (10 ng/ml) for 24 h. Total RNA was isolated and qPCR was run on the samples using Taqman primers/probes for IL21 R. Data is shown with SD for 2 independent experiments.

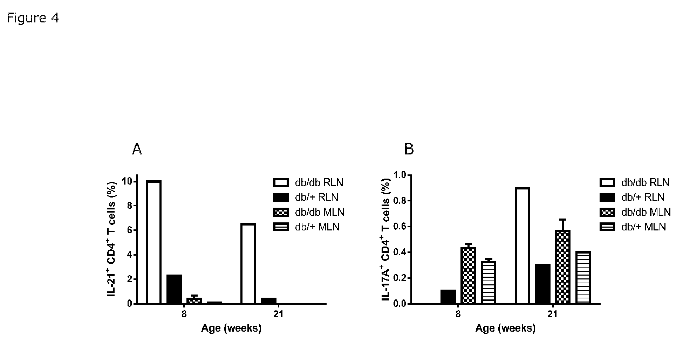

**Figure 4****:** Percentage of renal lymph node CD4⁺T cells expressing IL-21 and IL-17A. Renal (RLN) and mesenteric lymph nodes (MLN) were dissected from db/db and db/+ mice, and single cell suspensions were stimulated with PMA and lonomycin and stained intracellularly with antibodies against IL-21 and IL-17A. Data shows the percentage of CD4⁺ T cells expressing A. IL-21 and B. IL-17A (mean for RLN; mean and SEM of n=3 for MLN).

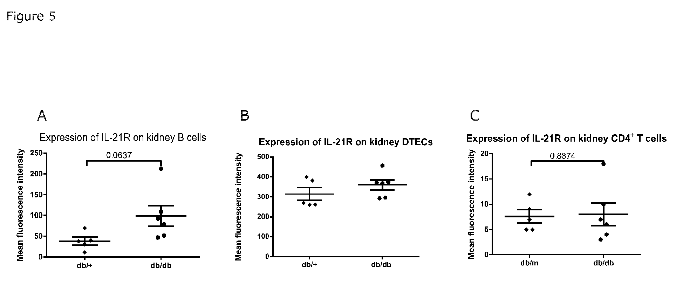

**Figure 5****:** Expression of IL21R in mouse kidney cells. Total kidney cell suspensions was prepared from db/db and db/+ mouse kidneys. Cells were stained with antibodies against CD45, B220, CD4, ENaC and IL-21R. Data is shown as mean and SEM of mean fluorescence intensity of IL-21 R staining on A. B cells, B. Distal tubular epithelial cells and C. T cells.

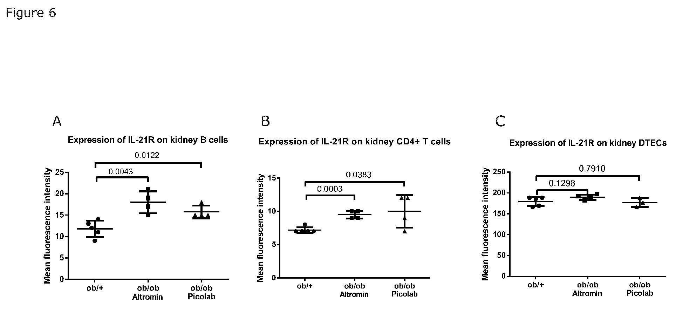

**Figure 6****:** Expression of IL21R in mouse kidney cells. Total kidney cell suspensions was prepared from BTBR ob/ob and BTBR ob/+ mouse kidneys. BTBR ob/ob mice were fed two different diets (Altromin and Picolab diets) for 12 weeks. Cells were stained with antibodies against CD45, B220, CD4, ENaC and IL-21 R. Data is shown as mean and SEM of mean fluorescence intensity of IL-21 R staining on A. B cells, B. T cells and C. Distal tubular epithelial cells.

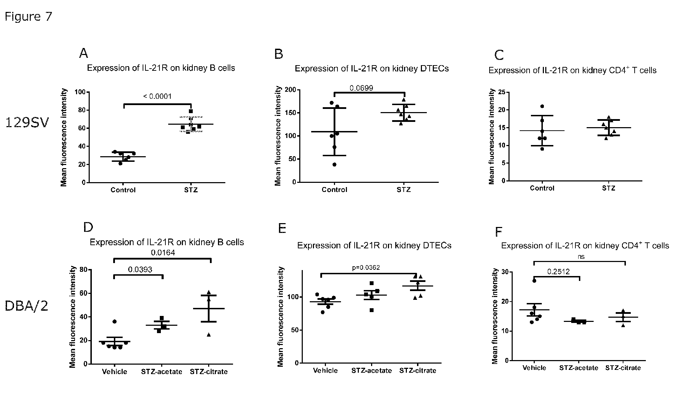

**Figure 7****:** Expression of IL21R in mouse kidney cells. Total kidney cell suspensions was prepared from mice treated with STZ and control mice 15 weeks after STZ treatment. Two different background strains of mice were used (129SV, A-C) and DBA/2, D-F). STZ was administered to DBA/2 mice in two different injection buffers. Cells were stained with antibodies against CD45, B220, CD4, ENaC and IL-21 R. Data is shown as mean and SEM of mean fluorescence intensity of IL-21 R staining on A. and D. B cells; B. and E. Distal tubular epithelial cells; C. and F. T cells.

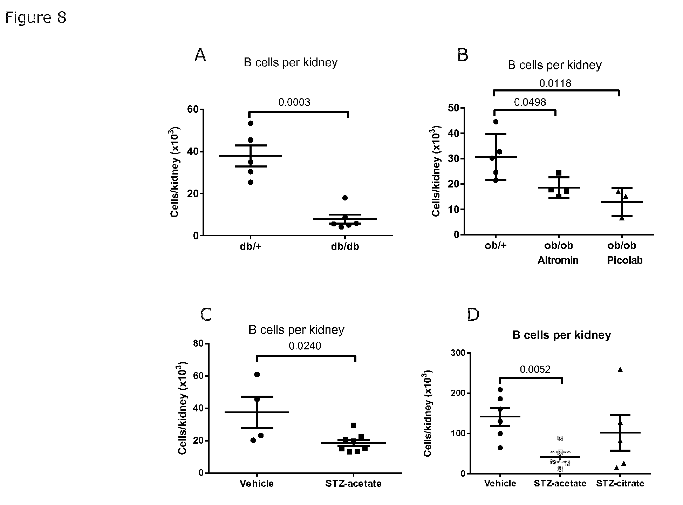

**Figure 8****:** Total number of B cells in kidneys of diabetic nephropathy mouse models. Total kidney cell suspensions was prepared from mouse kidneys with either genetically inherent (A and B) or STZ induced diabetes (C and D). BTBR ob/ob mice were fed two different diets (Altromin and Picolab diets) for 12 weeks. CD45 and B220. Data is shown as mean and SEM of B cells per kidney in A. db/db mice, B. BTBR ob/ob mice, C. STZ treated 129SV mice and D. STZ treated DBA/2 mice (STZ was administered in 2 different buffers).

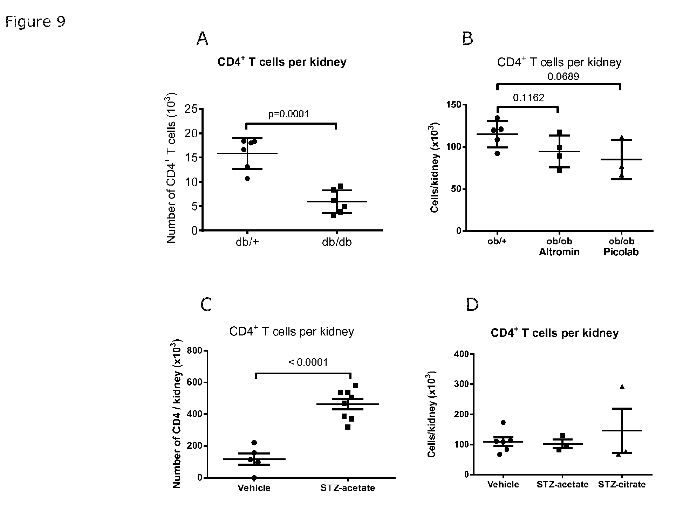

**Figure 9****:** Total number of CD4⁺ T cells in kidneys of diabetic nephropathy mouse models. Total kidney cell suspensions was prepared from mouse kidneys with either genetically inherent (A and B) or STZ induced diabetes (C and D). BTBR ob/ob mice were fed two different diets (Altromin and Picolab diets) for 12 weeks. CD45 and CD4. Data is shown as mean and SEM of B cells per kidney in A. db/db mice, B. BTBR ob/ob mice, C. STZ treated 129SV mice and D. STZ treated DBA/2 mice (STZ was administered in 2 different buffers).

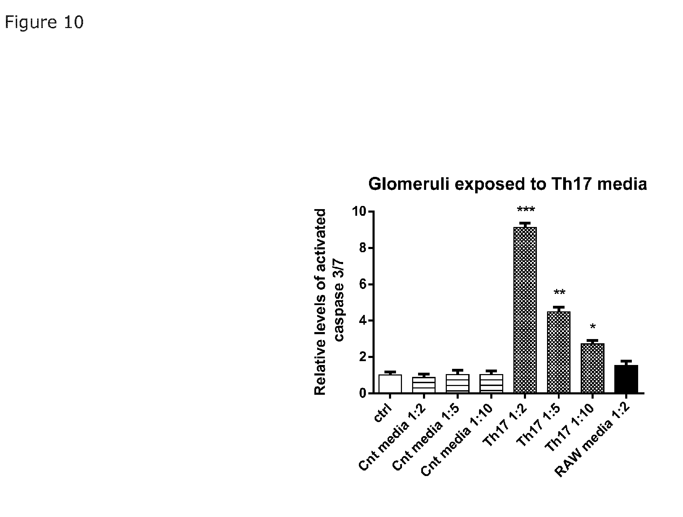

**Figure 10****:** Th17 media induces apoptosis in mouse glomeruli. Mouse glomeruli were isolated using magnetic Dynalbeads, perfused into the kidney, digested with collagenase and isolated using a magnetic particle concentrator. Glomeruli were acclimitized overnight and then treated with control media (50, 20, 10 %), Th17 media (50, 20 , 10 %) or RAW conditioned media (50 %) for 24 h. Relative levels of caspase 3 activity was measured using ApoTox-Glo Triplex assay (Promega). Data is shown with SEM from 3 independent experiments. * p<0.05, ** p<0.01, *** p<0.001.

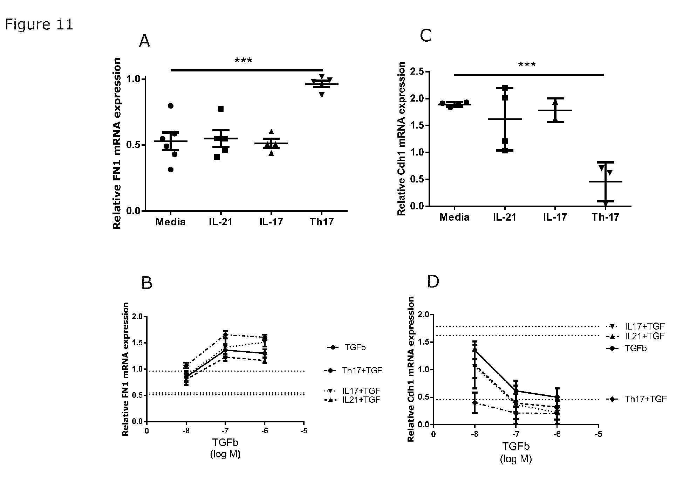

**Figure 11****:** Th17 media induces fibronectin and decreases E-cadherin mRNA expression. Human proximal tubular epithelial cells were treated with IL21 (200 ng/ml), IL17 (200 ng/ml) or Th17 media (10 %) for 48 h. Total RNA was isolated and analyzed by qPCR with Taqman primer/probes for fibronectin, FN1, and E-cadherin, Cdh1. Data is shown with SEM from 3-4 independent experiments. *** p<0.001.

### Definitions:

Causes of "nephropathy" include deposition of the IgA antibodies in the glomerulus, administration of analgesics, xanthine oxidase deficiency, and long-term exposure to lead or its salts. Chronic conditions that can produce nephropathy include systemic lupus erythematosus, diabetes mellitus and high blood pressure (hypertension), which lead to diabetic nephropathy and hypertensive nephropathy, respectively. Other types of nephropathy of relevance to the present invention include hypertensive nephropathy, interstitial nephritis; IgA-induced nephropathy; Goodpasture's syndrome; hemolytic-uremic syndrome; and glomerulonephritis;

"Diabetic nephropathy" (also known as Kimmelstiel-Wilson syndrome, or nodular diabetic glomerulo-sclerosis, or intercapillary glomerulonephritis) is a common chronic complication seen in patients with chronic diabetes. Diabetic nephropathy usually manifests 15-25 years after diagnosis of diabetes and affects 25-35% of patients under the age of 30 years. It is the leading cause of premature death in diabetic patients between 50 and 70 years olds. The disease is progressive and may cause death two or three years after the initial lesions. Diabetic nephropathy is the most common cause of chronic kidney failure and end-stage kidney disease in the Western world.

The risk of developing diabetic nephropathy is higher if blood-glucose levels are poorly controlled. Diabetes patients are suspected to have diabetic nephropathy if they have elevated levels of protein in the urine (proteinuria).

The earliest detectable kidney change in the course of diabetic nephropathy is a thickening in the glomerulus. At this stage, the kidney may leak more serum albumin (plasma protein) than normal in the urine ("albuminuria"), and this can be detected by sensitive medical tests for albumin. This stage is called "micro-albuminuria". As diabetic nephropathy progresses, increasing numbers of glomeruli are destroyed by progressive nodular glomerulo-sclerosis. Consequently, urine albumin increases to the point that it may be detected by ordinary urinalysis techniques. At this stage, a kidney biopsy generally clearly shows diabetic nephropathy.

The term "treatment", as used herein, refers to the medical therapy of any human or other animal subject in need thereof. Treatment of e.g. diabetic nephropathy may be prophylactic, palliative, symptomatic and/or curative.

As used herein, an "isolated" compound is a compound that has been removed from its natural environment. "A purified" compound is a compound that has been increased in purity, such that it exists in a form that is more pure than it exists in its natural environment.

The term "antibody", "recombinant antibody", "monoclonal antibody" and "mAb" as used herein, is intended to refer to immunoglobulin molecules and fragments thereof according to the invention that have the ability to specifically bind to an antigen. Full-length antibodies comprise four (or more) polypeptide chains, i.e. at least two heavy (H) chains and at least two light (L) chains interconnected by disulfide bonds. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hyper-variability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. Variable regions and CDRs in an antibody sequence may be identified by aligning the sequences against a database of known variable regions (frameworks and CDRs are defined according to the Kabat numbering scheme herein - (Kabat, EA, Wu, TT, Perry, HM, et al. Sequences of Proteins of Immunological Interest, Fifth Edition. US Department of Health and Human Services, Public Health Service, National Institutes of Health, NIH Publication No. 91-3242, 1991).

The fragment crystallizable region ("Fc region"/"Fc domain") of an antibody comprises the tail regions of an antibody that interact with cell surface receptors called Fc receptors and some proteins of the complement system. This property allows antibodies to activate the immune system. The Fc domain can, however, comprise amino acid mutations that result in modification of these effector functions. Preferably, a modified Fc domain comprises one or more, preferably all of the following mutations that will result in decreased affinity to certain Fc receptors (L234A, L235E, and G237A) and in reduced C1q-mediated complement fixation (A330S and P331 S), respectively (residue numbering according to the EU index). Such Fc domains will still retain a long *in vivo* circulatory half life.

The other part of an antibody, called the "Fab region"/"Fab domain"/"Fab fragment", contains variable regions that define the specific target that the antibody can bind. Fab fragments can be produced from intact antibodies using well known methods, for example by proteolytic cleavage with enzymes such as papain (to produce Fab fragments) or pepsin (to produce F(ab')₂ fragments). Alternatively, antibody fragments may be produced recombinantly, using standard recombinant DNA and protein expression technologies.

Examples of binding fragments encompassed within the term "antibody" thus include but are not limited to: (i) a Fab fragment, a monovalent fragment consisting of the VL, VH, CL and CH1 domains; (ii) F(ab)2 and F(ab')2 fragments, a bivalent fragment comprising two Fab fragments linked by a disulfide bridge at the hinge region; (iii) a Fd fragment consisting of the VH and CH1 domains; (iv) a scFv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423-426: and Huston et al. (1988) Proc. Natl. Acad. Sci. USA 85:5879-5883). Such single chain antibodies are also intended to be encompassed within the term "antibody". Other forms of single chain antibodies, such as diabodies are also encompassed within the term "antibody". Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see e.g., Hol-liger, P., et al. (1993) Proc. Natl. Acad. Sci. USA 90:6444-6448; Poljak, R. J., et al. (1994) Structure 2:1121-1123).

It is understood that the antigen may have one or more epitopes comprising (1) inants which consist of single peptide chains, (2) conformational epitopes comprising one or more spatially contiguous peptide chains whose respective amino acid sequences are located disjointedly along polypeptide sequence; and (3) post-translational epitopes which comprise, either in whole or part, molecular structures covalently attached to the antigen after translation, such as carbohydrate groups, or the like.

The terms "human antibody", "human antibodies", as used herein, means antibodies having variable and constant regions derived from human germline immunoglobulin sequences. The human antibodies of the invention may include amino acid residues not encoded by human germline immunoglobulin sequences (e.g., mutations introduced by random or site-specific mutagenesis in vitro or by somatic mutation in vivo), for example in the CDRs and in particular CDR3.

Antibodies in which CDR sequences derived from antibodies originating from another mammalian species (such as e.g. a mouse), have been grafted onto human framework sequences and optionally potentially further engineered by mutagenesis are referred to as "humanized antibodies".

The term "chimeric antibody" or "chimeric antibodies" refers to antibodies according to the invention where the light and heavy chain genes have been constructed, typically by genetic engineering, from immunoglobulin variable and constant region genes belonging to different species. For example, the variable segments of genes from a mouse monoclonal antibody may be joined to human constant segments.

The term "epitope", as used herein, is defined in the context of a molecular interaction between an "antigen binding polypeptide", such as an antibody (Ab), and its corresponding antigen (Ag). Generally, "epitope" refers to the area or region on an Ag to which an Ab specifically binds, i.e. the area or region in physical contact with the Ab. Physical contact may be defined through various criteria (e.g. a distance cut-off of 2-6A, such as 3A, such as 4 A, such as 5A; or solvent accessibility) for atoms in the Ab and Ag molecules. A protein epitope may comprise amino acid residues in the Ag that are directly involved in binding to a Ab (also called the immuno-dominant component of the epitope) and other amino acid residues, which are not directly involved in binding, such as amino acid residues of the Ag which are effectively blocked by the Ab, i.e. amino acid residues within the "solvent-excluded surface" and/or the "footprint" of the Ab.

The epitope for a given antibody (Ab)/antigen (Ag) pair can be described and characterized at different levels of detail using a variety of experimental and computational epitope mapping methods. The experimental methods include mutagenesis, X-ray crystallography, Nuclear Magnetic Resonance (NMR) spectroscopy, Hydrogen deuterium eXchange Mass Spectrometry (HX-MS) and various competition binding methods; methods that are known in the art. As each method relies on a unique principle, the description of an epitope is intimately linked to the method by which it has been determined. Thus, depending on the epitope mapping method employed, the epitope for a given Ab/Ag pair may be described differently.

Antibodies that bind to the same antigen can be characterised with respect to their ability to bind to their common antigen simultaneously and may be subjected to "competition binding"/"binning". In the present context, the term "binning" refers to a method of grouping antibodies that bind to the same antigen. "Binning" of antibodies may be based on competition binding of two antibodies to their common antigen in assays based on standard techniques such as surface plasmon resonance (SPR), ELISA or flow cytometry.

An antibody's "bin" is defined using a reference antibody. If a second antibody is unable to bind to an antigen at the same time as the reference antibody, the second antibody is said to belong to the same "bin" as the reference antibody. In this case, the reference and the second antibody competitively bind the same part of an antigen and are coined "competing antibodies". If a second antibody is capable of binding to an antigen at the same time as the reference antibody, the second antibody is said to belong to a separate "bin". In this case, the reference and the second antibody do not competitively bind the same part of an antigen and are coined "non-competing antibodies".

A "neutralizing" antibody according to the invention is an antibody having the ability to significantly inhibit/reduce signalling through the IL-21Rα. Neutralizing effects can be assessed in various functional assays using cells expressing IL-21Rα and γC, e.g. B cell proliferation assays as disclosed in WO2012098113.

The term "affinity", as used herein, defines the strength of the binding of an antibody to an epitope. The affinity of an antibody is measured by the equilibrium dissociation constant KD, defined as [Ab] x [Ag] / [Ab-Ag] where [Ab-Ag] is the molar concentration of the antibody-antigen complex, [Ab] is the molar concentration of the unbound antibody and [Ag] is the molar concentration of the unbound antigen at equilibrium. KD can also be described from the kinetics of complex formation and dissociation, determined by e.g. the SPR method. The rate constants corresponding to the association and the dissociation of a monovalent complex are referred to as the association rate constant ka (or kₒₙ) and dissociation rate constant kd (or k_{off}), respectively. KD is then related to ka and kd through the equation KD = kd / ka. The affinity constant KA is defined by 1/KD. Preferred methods for determining antibody specificity and affinity by competitive inhibition can be found in Harlow, et al., Antibodies: A Laboratory Manual, Cold Spring Harbor Laboratory Press, Cold Spring Harbor, N.Y., 1988), Colligan et al., eds., Current Protocols in Immunology, Greene Publishing Assoc.and Wiley Interscience, N.Y., (1992, 1993), and Muller, Meth. Enzymol. 92:589-601 (1983). Use of high affinity antibodies are preferred in connection with the present invention, e.g. antibodies binding specifically to the antigen with a binding affinity as measured by e.g. SPR of e.g. 10⁷ M⁻¹ or greater, 10⁸ M⁻¹ or greater, 10⁹ M⁻¹ or greater, 10¹⁰ M⁻¹ or greater, 10¹¹ M⁻¹ or greater, or 10¹² M⁻¹ or greater.

The antibody or fragment thereof may be modified in order to increase its serum half-life, for example, by conjugating with "half life extending moieties" - such as fatty acids or fatty acid derivates, PEG (poly ethylene glycol) or other water soluble polymers, including polysaccharide polymers and peptide derived polymers to increase the half-life. "Half life extending moieties" may also be in the form of peptides, preferably in the form of fusion proteins. Half life extending moieties conjugated to antagonists according to the invention thus include e.g. albumin fused to antibodies and Fc domains fused to antibodies, or fragments thereof, according to the invention. Fusion protein s are preferably made by recombinant techniques.

"Biological drugs"/"biologics" can be composed of sugars, proteins, or nucleic acids or complex combinations of these substances. Biological drugs are isolated from a variety of natural sources (rather than being chemically synthesized) - human, animal, or microorganism - and may be produced by biotechnology methods and other technologies. Biological drugs according to the present invention are preferably derived from recombinant proteins that may optionally be subject to post-translational modification, such as e.g. conjugation with polymeric compounds and or processing to yield fragments of said recombinant proteins (e.g. processing of an antibody to Fab fragments).

"IL-21" has a four helix bundle structure (helix 1-4/A-D - (helix 1-4/A-D - as defined e.g. in Figure 1 in: J. Immunol., 2011 vol. 186 no. 2, p. 708-721)), arranged in an up-up-down-down topology typical for the class I cytokines. IL-21 signals through a heterodimeric receptor complex, consisting of the private chain IL-21 Rα (also referred to as IL-21 R) and the γC/IL-2Ry/common gamma chain the latter being shared by IL-2, IL-4, IL-7, IL-9, and IL-15. γC and IL-21Rα bind to non-overlapping binding sites on IL-21 - IL-21Rα binds to helix 1+3 and γC binds to helix 2+4 on human IL-21. IL-21 Rα binds IL-21 with high affinity and provides the majority of the binding energy. However, interaction with γC is required for signaling and IL-21 mutants which bind IL-21Rα but fail to interact properly with γC are potent antagonists of IL-21 signaling. IL-21 exerts pleiotropic effects on both innate and adaptive immune responses. It is mainly produced by activated CD4+ T cells, follicular T cells and Natural killer cells. The amino acid sequence of immature human IL-21 is shown in **SEQ ID NO 1.**

IL-21 contributes to inflammation mainly through actions mediated by leukocytes expressing IL-21Rα and γC. It is also reported to potentially contribute to enhanced fibrosis under chronic inflammatory conditions. Patients suffering from diabetic nephropathy have elevated inflammatory responses locally in the kidney (enhanced levels of pro-inflammatory cytokines like IL-21) and suffer from extensive fibrosis. The proximal tubular epithelial cells (PTEC) express γC and are located at sites of massive fibrosis. These factors together indicate that IL-21 induced signalling plays a role in diabetic nephropathy.

The inventors have herein shown that IL-21 Rα is expressed on the surface of proximal tubular epithelial cells in diabetic mice, indicating that these cells are sensitive to IL-21. The inventors have furthermore shown that IL-21/IL-17 may be involved in apoptosis of glomeruli cells in diabetic mice. Diabetic mice also have increased amount of IL-21 present in their draining kidney lymph nodes. These findings indicate that IL-21 antagonists such as e.g. IL-21 antibodies with neutralizing/antagonistic effects may decrease the rate of glomerulus apoptosis and/or decrease the degree of kidney inflammation in diabetic nephropathy patients. Kidneys from human diabetic nephropathy patients are furthermore populated with an increased number of inflammatory cells (leukocytes, monocytes, macrophages, dendritic cells, neutrophils, etc.), and neutralizing IL-21 and/or IL-21 Rα antibodies thus appear to be an attractive drug type for treatment of diabetic nephropathy in humans either alone or in combination with e.g. ACE inhibitors or other drugs.

IL-21/IL-21Rα antagonists according to the invention are agents with the ability to disrupt/inhibit/reduce IL-21 mediated signalling/effects. Preferred IL-21 Rα antagonists according to the present invention are neutralizing IL-21 antibodies having the ability to compete with either γC or the IL-21 Rα for binding to IL-21.

An example of an IL-21 antibody competing with IL-21 Rα for binding to IL-21 is the "mAb 5" antibody which is a human antibody first disclosed in WO2010055366 as clone number 362.78.1.44. The amino acid sequences of mAb 5 heavy and light chains are shown in **SEQ ID NOs 2+3** (IgG1 isotype version). The mAb 5 antibody binds to helix 1+3 of human IL-21, or more specifically amino acids I37 to Y52 and N92 to P108, as set forth in SEQ ID NO 1. The binding properties of mAb 5, and variants thereof, and their advantages (high affinity and potency), is described in greater detail in WO2012098113. JBC, VOL. 287, NO. 12, pp. 9454-9460, March 16, 2012 discloses further details about IL-21/IL-21Rα binding.

An example of an IL-21 antibody competing with γC for binding to IL-21 is the "mAb 14" antibody which is a human antibody first disclosed in WO2010055366 as clone number 366.328.10.63. "mAb" 14 binds to helix 2+4 of human IL-21. More specifically, mAb 14 binds to Glu 65, Asp 66, Val 67, Glu 68, Thr 69, Asn 70, Glu 72, Trp 73, Lys 117, His 118, Arg 119, Leu 143, Lys 146, Met 147, His 149, Gln 150, and His 151 of human IL-21. The amino acid sequences of mAb 14 heavy and light chains are shown in **SEQ ID NOs 4+5.**

mAb 5 and mAb 14 type of antibodies are generally characterized by having an unusually high affinity (in the nanomolar range) to IL-21 and high potency to neutralize IL-21 induced effects.

Preferred (neutralizing) IL-21 Rα antagonists according to the present invention are those that compete with IL-21 for binding to IL-21 Rα. IL-21 Rα antagonists according to the invention are preferably antibodies preferably bind the loops connecting the β-strand. IL-21Rα antibodies according to the invention bind to the EF loop. Preferred IL-21 Rα antibodies according to the invention bind Tyr 29, Gln 52, Gln 54, Tyr 55, Glu 57, Leu 58, Phe 86, His 87, Phe 88, Met 89, Ala 90, Asp 91, Asp 92, Ile 93, Leu 113, Ala 115, Pro 145, Ala 146, Tyr 148, Met 149, Lys 153, Ser 209, Tyr 210 of IL-21Rα (SEQ ID NO 6).

Preferred IL-21/IL-21Rα antagonists according to the invention are IL-21Rα antibodies that intefrere with binding of IL-21Rα with γC and thus assembly of the IL-21/IL-21Rα/γC complex.

An "ACE inhibitor" (or angiotensin-converting-enzyme inhibitor) is a pharmaceutical drug used primarily for the treatment of hypertension (high blood pressure) and congestive heart failure. Frequently prescribed ACE inhibitors include perindopril, captopril, enalapril, lisinopril, and ramipril. ACE inhibitors are most frequently administered orally.

An IL-21/IL-21Rα antagonist of the invention may be administered parenterally (e.g. intravenously, intramuscularly, subcutaneously or intradermally) and prophylactically and/or therapeutically (on demand).

IL-21/IL-21Rα antagonists according to the invention may be "co-administered" with one or more other therapeutic agents. The other agent(-s) may be an agent that enhances the effects of the IL-21/ILRα antagonist. The other agent(-s) may be intended to treat other symptoms or conditions of the patient. For example, the other agent(-s) may be an ACE inhibitor, an analgesic, an immunosuppressant (e.g. methotrexate, dexamethasone, and/or prednisone) or an anti-inflammatory agent. In addition, diabetic nephropathy patients preferably also receive treatment with insulin or insulin derivatives/variants/analogues and/or one or more other anti-diabetic medicines such as e.g. metformin, DPP4 inhibitors, GLP-1, GLP-1 derivatives/variants/analogues, drugs that bind to ATP-dependent K channels on the cell membrane of pancreatic beta cells (sulfonylurea, meglitinides (such as e.g. repaglinide)) etc.

Combined administration of two or more agents may be achieved in a number of different ways. The IL-21/IL-21Rα antagonist and the other agent may be administered together in a single composition or in separate compositions as part of a combined therapy.

IL-21/IL-21Rα antagonists according to the invention may be produced by means of recombinant nucleic acid techniques. In general, a cloned wild-type nucleic acid sequence is modified to encode the desired protein. This modified sequence is then inserted into an expression vector, which is in turn transformed or transfected into host cells.

In another aspect, the present invention provides compositions and formulations comprising IL-21/IL-21Rα antagonists. For example, the invention provides a pharmaceutical composition that comprises one or more antagonists of the invention, formulated together with one or more pharmaceutically acceptable carrier. The use of preservatives, isotonic agents, chelating agents, stabilizers and surfactants in pharmaceutical compositions is well-known to the skilled person. In one embodiment, the pharmaceutical formulation is an aqueous formulation. The term "aqueous formulation" is defined as a formulation comprising at least 50% w/w water. In another embodiment, the pharmaceutical formulation is a freeze-dried formulation, to which the physician or the patient adds solvents and/or diluents prior to use. In a further aspect, the pharmaceutical formulation comprises an aqueous solution of such an antibody, and a buffer, wherein the antibody is present in a concentration from 1 mg/ml or above (such as e.g. 1-200, 1-100, 50-200, 50-150, 50-100, 1, 5, 10, 15, 20, 25, 30, 35, 40, 45, 50, 60, 70, 80, 90, 100, 125, 150, 175, or 200 mg/ml) and wherein said formulation has a pH from about 6.0 to about 8.0, such as e.g. about 6.0, 6.1, 6.2, 6.3, 6.3, 6.4, 6.5, 6.5, 6.6, 6.7, 6.8, 6.7, 6.8, 6.9, 7.0, 7.1, 7.2, 7.3, 7.4, 7.5, 7.6, 7.7, 7.8, 7.8, 7,9, or 8.0.

Compositions comprising compounds according to the invention are preferably parenteral formulations intended for either extravascular (such as e.g. subcutanous (s.c.) or intradermal) or intravenous (i.v.) administration.

### SEQUENCES:

**SEQ ID No 1:** hIL-21 (incl. signal peptide spanning amino acids 1-29 - mAb 5 epitope shown in bold; IL-21Rα binding site shown in underline; amino acid residues forming the mAb 14 epitope shown with lower case letters in italics) 
MRSSPGNMERIVICLMVIFLGTLVHKSSSQGQDRHMIRMRQLIDIVDQLKNYVNDL
VPEFLPAPedvetnCewSAFSCFQKAQLKSANTGNNERIINVSIKKLKRKPPSTNAGRRQkhrLT
CPSCDSYEKKPPKEFLERFKSlLQkmIhqhLSSRTHGSEDS
**SEQ ID No 2:** "mAb 5": light chain (signal peptide omitted - CDR sequences shown in bold/underline - constant region shown in lowercase letters) 
EIVLTQSPGTLSLSPGERATLSCRASQSVSSSYLAWYQQKPGQAPRLLIYGASSR
ATGIPDRFSGSGSGTDFTLTISRLEPEDFAVYYCQQYGSWTFGQGTKVEIKRtvaapsvfifppsd
eqlksgtasvvcllnnfypreakvqwkvdnalqsgnsqesvteqdskdstyslsstltlskadyekhkvyacevthqglsspvtksf
nrgec
**SEQ ID No 3:** "mAb 5"; heavy chain of the IgG1 isotype (signal peptide omitted - CDR sequences shown in bold/underline - constant region shown in lowercase letters) 
QVQLVESGGGVVQPGRSLRLSCAASGFTFSSYGMHWVRQAPGKGLEWVAFIWY
DGSDKYYADSVKGRFTISRDNSKNTLYLQMNSLRAEDTAVYYCARDGDSSDWYGDYYFG
MDVWGQGTTVTVSSastkgpsvfplapsskstsggtaalgclvkdyfpepvtvswnsgaltsgvhtfpavlqssglyslss
vvtvpssslgtqtyicnvnhkpsntkvdkkvepkscdkthtcppcpapeaegapsvflfppkpkdtlmisrtpevtcvvvdvshe
dpevkfnwyvdgvevhnaktkpreeqynstyrvvsvltvlhqdwlngkeykckvsnkalpssiektiskakgqprepqvytlpps
rdeltknqvsltclvkgfypsdiavewesngqpennykttppvldsdgsfflyskltvdksrwqqgnvfscsvmhealhnhytqksl
slspgk
**SEQ ID No 4:** "mAb14" light chain (signal peptide omitted - CDR sequences shown in bold/underline, constant region shown in lowercase letters) 
AIQLTQSPSSLSASVGDRVTITCRASQDIDSALAWYQQKPGKAPKILIHDASSLESG
VPSRFSGSGSGTDFTLTISSLQPEDFATYYCQQFNSYPYTFGQGTKLEIKRtvaapsvfifppsdeq
 
lksgtasvvcllnnfypreakvqwkvdnalqsgnsqesvteqdskdstyslsstltlskadyekhkvyacevthqglsspvtksfnr
gec
**SEQ ID No 5:** "mAb14"; heavy chain of the IgG4 isotype (signal peptide omitted - CDR sequences shown in bold/underline, constant region shown in lowercase letters) 
EVQLVESGGGLVKPGGSLRLSCAASGFIFSSYSMNWVRQAPGKGLEWVSSITSGS
YYIHYADSVKGRFTISRDNAKNSLYLQMNSLRAEDTAVYYCVRERGWGYYGMDVWGQGT
TVTVSSastkgpsvfplapcsrstsestaalgclvkdyfpepvtvswnsgaltsgvhtfpavlqssglyslssvvtvpssslgtkty
tcnvdhkpsntkvdkrveskygppcpscpapeflggpsvflfppkpkdtlmisrtpevtcvvvdvsqedpevqfnwyvdgvevh
naktkpreeqfnstyrvvsvltvlhqdwlngkeykckvsnkglpssiektiskakgqprepqvytlppsqeemtknqvsltclvkgf
ypsdiavewesngqpennykttppvldsdgsfflysrltvdksrwqegnvfscsvmhealhnhytqkslslslgk
**SEQ ID No 6:** IL-21Rα (incl. signal sequence) 
MPRGWAAPLLLLLLQGGWGCPDLVCYTDYLQTVICILEMWNLHPSTLTLTWQDQY
EELKDEATSCSLHRSAHNATHATYTCHMDVFHFMADDIFSVNITDQSGNYSQECGSFLLAE
SIKPAPPFNVTVTFSGQYNISWRSDYEDPAFYMLKGKLQYELQYRNRGDPWAVSPRRKLIS
VDSRSVSLLPLEFRKDSSYELQVRAGPMPGSSYQGTWSEWSDPVIFQTQSEELKEGWNPH
LLLLLLLVIVFIPAFWSLKTHPLWRLWKKIWAVPSPERFFMPLYKGCSGDFKKWVGAPFTGS
SLELGPWSPEVPSTLEVYSCHPPRSPAKRLQLTELQEPAELVESDGVPKPSFWPTAQNSG
GSAYSEERDRPYGLVSIDTVTVLDAEGPCTWPCSCEDDGYPALDLDAGLEPSPGLEDPLLD
AGTTVLSCGCVSAGSPGLGGPLGSLLDRLKPPLADGEDWAGGLPWGGRSPGGVSESEAG
SPLAGLDMDTFDSGFVGSDCSSPVECDFTSPGDEGPPRSYLRQWVVIPPPLSSPGPQAS
**SEQ ID No 7:** γC (Common gamma chain/IL-2Rγ) incl. signal sequence

### LIST OF EMBODIMENTS:

The present invention is further exemplified in the embodiments below - none of which should be construed in a limiting way.
1. An IL-21/IL-21Rα antagonist for use in treating nephropathy, optionally selected from the list consisting of: diabetic nephropathy, hypertensive nephropathy, interstitial nephritis; IgA-induced nephropathy; Goodpasture's syndrome; hemolytic-uremic syndrome; and glomerulonephritis. Preferably, inflammatory nephropathy (nephritis).
2. An IL-21/IL-21Rα antagonist for use in treating diabetic nephropathy.
3. An IL-21/IL-21Rα antagonist for use in treating diabetic hyper-filtration, optionally diabetic hyper-filtration in combination with diabetes and/or diabetic nephropathy.
4. An IL-21/IL-21Rα antagonist for use in treating renal hypertrophy, optionally renal hypertrophy in combination with diabetes and/or diabetic nephropathy.
5. An IL-21/IL-21Rα antagonist for use in treating micro-albuminuria, optionally micro-albuminuria in combination with diabetes and/or diabetic nephropathy.
6. An IL-21/IL-21Rα antagonist for use in treating albuminuria, optionally albuminuria in combination with diabetes and/or diabetic nephropathy.
7. An IL-21/IL-21Rα antagonist for use in treating hypertension, optionally hypertension in combination with diabetes and/or diabetic nephropathy.
8. An IL-21/IL-21Rα antagonist for use in treating proteinuria, optionally proteinuria in combination with diabetes and/or diabetic nephropathy.
9. An IL-21/IL-21Rα antagonist for use in treating end-stage renal disease, optionally end-stage renal disease in combination with diabetes and/or diabetic nephropathy.
10. An IL-21/IL-21Rα antagonist for use in treating glomerulo-sclerosis, optionally glomerulo-sclerosis in combination with diabetes and/or diabetic nephropathy.
11. An IL-21/IL-21Rα antagonist for use in treating hypertensive nephropathy, optionally hypertensive nephropathy in combination with diabetes and/or diabetic nephropathy.
12. An IL-21/IL-21Rα antagonist for use in treating interstitial nephritis, optionally interstitial nephritis in combination with diabetes and/or diabetic nephropathy.
13. An IL-21/IL-21Rα antagonist for use in treating IgA-induced nephropathy, optionally IgA-induced nephropathy in combination with diabetes and/or diabetic nephropathy.
14. An IL-21/IL-21Rα antagonist for use in treating Goodpasture's syndrome, optionally Goodpastures syndrome in combination with diabetes and/or diabetic nephropathy.
15. An IL-21/IL-21Rα antagonist for use in treating hemolytic-uremic syndrome, optionally hemolytic-uremic syndrome in combination with diabetes and/or diabetic nephropathy.
16. An IL-21/IL-21Rα antagonist for use in treating glomerulo-nephritis, optionally glomerulo-nephritis in combination with diabetes and/or diabetic nephropathy.
17. An IL-21/IL-21Rα antagonist for use in treating diabetic nephropathy in patients that are 30 years old or above or 30 years old and below.
18. An IL-21/IL-21Rα antagonist for use in treating diabetic nephropathy in patients that are 50 years old or above or 50 years old and below.
19. An IL-21/IL-21Rα antagonist for use in treating diabetic nephropathy in patients that are about 30 to 50 years old.
20. An IL-21/IL-21Rα antagonist for use in treating diabetic nephropathy in patients that have had diabetes for at least 15, 20, or 25 years.
21. An IL-21/IL-21Rα antagonist according to the invention, wherein said antagonist is a biological drug.
22. An IL-21/IL-21Rα antagonist according to the invention, wherein said biological drug is a neutralizing IL-21/IL-21Rα antibody.
23. An antibody according to the invention, wherein said antibody is an IL-21 antibody that competes with IL-21 Rα for binding to IL-21.
24. An IL-21 antibody according to the invention, wherein said antibody competes with IL-21Rα for binding to IL-21, wherein said antibody binds to helix 1 +3 of human IL-21.
25. An IL-21 antibody according to the invention, wherein said antibody wherein said antibody competes with IL-21 Rα for binding to IL-21 and binds to a discontinuous epitope on IL-21, wherein said epitope comprises amino acids I37 to Y52 and N92 to P108 as set forth in SEO ID NO 1.
26. An IL-21 antibody according to the invention, wherein said antibody comprises the three CDR sequences as set forth in SEQ ID NO 2 and the three CDR sequences as set forth in SEQ ID NO 3.
27. An IL-21 antibody according to the invention, wherein said antibody comprises at least one (e.g. 2, 3, 4, 5, 6, 7, 8, 9, or 10) substitution/deletions/insertions in at least one (e.g. 2, 3, 4, 5, or 6) of the 6 CDR sequences as set forth in SEQ ID NO 2 and SEO ID NO 3..
28. An IL-21 antibody according to the invention, wherein said antibody comprises the VL and the VH sequences as set forth in SEQ ID NO 2 and SEQ ID NO 3.
29. An IL-21 antibody according to the invention, wherein said antibody comprises the heavy and light chain sequences as set forth in SEQ ID NO 2 and SEQ ID NO 3.
30. An IL-21 antibody according to the invention, wherein said antibody is an IL-21 antibody that competes with γC for binding to IL-21.
31. An IL-21 antibody according to the invention, wherein said antibody binds to helix 2+4 of human IL-21.
32. An IL-21 antibody according to the invention, wherein said antibody binds to an epitope comprising amino acids Glu 65, Asp 66, Val 67, Glu 68, Thr 69, Asn 70, Glu 72, Trp 73, Lys 117, His 118, Arg 119, Leu 143, Lys 146, Met 147, His 149, Gln 150, and His 151 as set forth in SEQ ID NO.1
33. An IL-21 antibody according to the invention, wherein said antibody comprises the three CDR sequences as set forth in SEQ ID NO 4 and the three CDR sequences as set forth in SEQ ID NO 5.
34. An IL-21 antibody according to the invention, wherein said antibody comprises at least one (such as e.g. 2, 3, 4, 5, 6, 7, 8, 9, or 10) substitutions/deletions/insertions in at least one ( e.g. 2, 3, 4, 5, or 6) of the 6 CDR sequences as set forth in SEQ ID NO 4 and SEQ ID NO 5.
35. An IL-21 antibody according to the invention, wherein said antibody comprises the VL and the VH sequence as set forth in SEQ ID NO 4 and SEQ ID NO 5.
36. An IL-21 antibody according to the invention, wherein said antibody comprises the heavy and light chains as set forth in SEQ ID NO 4 and SEQ ID NO 5.
37. An antibody according to the invention, wherein said antibody is a (neutralizing) IL-21Rα antibody that competes with IL-21 for binding to IL-21 Rα.
38. An IL-21 Rα antibody according to the invention, wherein said antibody binds to an epitope comprising amino acids Tyr 29, Gln 52, Gln 54, Tyr 55, Glu 57, Leu 58, Phe 86, His 87, Phe 88, Met 89, Ala 90, Asp 91, Asp 92, Ile 93, Leu 113, Ala 115, Pro 145, Ala 146, Tyr 148, Met 149, Lys 153, Ser 209, Tyr 210 as set forth in SEQ ID NO 6.
39. An antibody according to the invention, wherein said antibody specifically binds to IL-21 with a binding affinity of 10⁷ M⁻¹ or greater.
40. An antibody according to the invention, wherein said antibody specifically binds to IL-21 with a binding affinity of 10⁸ M⁻¹ or greater.
41. An antibody according to the invention, wherein said antibody specifically binds to IL-21 with a binding affinity of 10⁹ M⁻¹ or greater.
42. An antibody according to the invention, wherein said antibody specifically binds to IL-21 with a binding affinity of 10¹⁰ M⁻¹ or greater.
43. An antibody according to the invention, wherein said antibody specifically binds to IL-21 with a binding affinity of 10¹¹ M⁻¹ or greater.
44. An antibody according to the invention, wherein said antibody specifically binds to IL-21 with a binding affinity of 10¹² M⁻¹ or greater.
45. An antibody according to the invention, wherein said antibody is co-administered with one or more ACE inhibitors.
46. An antibody according to the invention, wherein said antibody is co-administered with insulin or an insulin derivative/analogue.
47. An antibody according to the invention, wherein said antibody is co-administered with metformin.
48. An antibody according to the invention, wherein said antibody is co-administered with a dipeptidyl peptidase-4 inhibitor (DPP-4 inhibitor) such as e.g. sitagliptin.
49. An antibody according to the invention, wherein said antibody is co-administered with GLP-1 or a GLP-1 derivative/analogue, such as e.g. liraglutide.
50. An antibody according to the invention, wherein said antibody is co-administered with a meglitinide such as e.g. repaglinide.
51. A pharmaceutical composition comprising an IL-21/IL-21Rα antagonist according to the invention (preferably an antibody) for use in treating diabetic nephropathy.
52. A pharmaceutical composition according to the invention, wherein said composition is an aqueous composition. Preferably a parenteral composition. Preferably for extravascular (e.g. subcutaneous or intradermal) or i.v.administration.
53. A pharmaceutical composition according to the invention, wherein said composition comprises at least one pharmaceutically acceptable carrier.
54. An IL-21/IL-21Rα antagonist according to the invention for manufacture of a medicament for treatment of diabetic nephropathy.
55. A method of treating diabetic nephropathy, wherein said method comprises administration to a patient in need thereof an appropriate amount of an IL-21/IL-21Rα antagonist.
56. A method of treatment according to the invention, wherein said IL-21/IL-21Rα antagonist is a (neutralizing) IL-21 antibody that competes with IL-21 Rα for binding to IL-21.
57. A method of treatment according to the invention, wherein said antagonist is a (neutralizing) IL-21 antibody that competes with γC for binding to IL-21.

### EXAMPLES

### Materials and methods

### Cell line culture

Murine podocyte SVI immortalized cells (Cell Lines Service, Eppelheim, Germany), human primary mouse proximal tubule epithelial cells (PTECs, ScienCell M4100) and primary glomerular endothelial cells (Applied Cell Biology Research Institute, Kirland, WA, US) were cultured according to the vendors' instructions.

### Th 17 mouse culture

Splenocytes from C57BI/6 mice were obtained by passage through a 70um mesh. The splenocytes were then labelled with magnetically labelled with CD4+ CD62L+T Cell Isolation Kit II (Miltenyi Biotec no. 130-093227) for 15min at 4C. The cells were then allowed to pass through a column in a magnetic field leaving the naïve CD4 positive cells trapped in the column. The column was then removed from the magnetic field and the cells were then eluded with PBS. These enriched CD4 T cells were then cultured in RPMI-1640 media supplemented with 10% fetal calf sera in T25-flasks coated with anti-CD3 (BD no. 553057). The media was supplemented with anti-CD28 (BD no. 553294), recombinant TGFb (R&D Systems), recombinant IL-6 (BD no. 554582), recombinant IL-1b (BD no. 554577), anti-IL-4 (&D Systems no. MAB404), anti-IFNg (R&D Systems no. MAB485) and IL-23 (BioLegend no. 589002). After four days in culture the T cells were polarized into Th17-phenotype and the Th17-media collected for future use.

### Th17 human culture

Human peripheral blood mononuclear cells (PBMCs) were prepared from peripheral blood of healthy donors by density gradient centrifugation. The PBMC were seeded in anti-CD3 coated 96-well plates at a cell density of 2x10⁶ cells / ml) in RPMI-1640 media supplemented with 10% fetal calf serum, 1 ug/ml anti-CD28, 10 ng/ml IL-6, 5 ng/ml recombinant TGFb1, 10 ng/ml recombinant IL-23, 10 ug/ml anti-IL-4 and 10 ug/ml anti-IFNγ. The plate were cultured for 14 days during which time the media was changed to fresh media every second day. At day 14, 50 ng/ml PMA and 1 ug/ml ionomycin was added to the cells for 6 hours. Plates were then spun down and Th17 media harvested.

### Renal lymph node stimulation

Db/db and db/+ mice on the C57BL/Ks background were sacrificed at 8 and 21 weeks of age and renal and mesenteric lymph nodes were dissected. The lymph nodes were passed through a 40 µm cell strainer and cultured for 4 hours in RPMI1640 supplemented with 10 % fetal calf serum, 100 units of penicillin and 100 ug of streptomycin (both Gibco), 20 ng/ml phorbol 12-myristate 13-acetate (Sigma) and 0.5 µg/ml ionomycin (Sigma). 10 µg/ml Brefeldin A (Cell Signalling) was added for the last 2 hours. Cells were harvested and stained with anti-CD4-Pacific blue (Biolegend, clone RM4-5). Cells were then fixed and permeabilized (Cytofix/Cytoperm, BD Biosciences) and stained with anti-IL21-PE (R&D Systems, clone 149204) and anti-IL-17A-AlexaFluor647 (Biolegend, clone TC11-18H10.1). The percentages of IL21- and IL17A-expressing cells were analysed on a BD Fortessa (BD Biosciences).

### RNA isolation from kidney cells and human cell lines and qRT-PCR

Total RNA from mouse kidney, mouse glomeruli, MES13 mouse mesangial cells, SVI40 mouse podocyte cells, human proximal tubular epithelial cells and human glomerular endothelial cells was isolated using trizol and the RNeasy kit (Qiagen, Denmark) and quantified on the NanoDrop 1000 micro-volume spectrophotometer. First strand cDNA was synthesized from 1.5 µg of RNA using the High Capacity cDNA Reverse Transcription Kit (Applied Biosystems, CA, USA) following the manufacture's protocol. Real time RT-PCR was performed on custom Taqman 384-well microfluidic cards using 50 µl 2x Taqman Universal Master Mix (Applied Biosystems) and 50 µ template (approximately 1500 ng) and run on the Applied Biosystems Prism 7900HT real-time PCR machine for 40 cycles. The results were analysed using RQ Manager 1.2.1 (Applied Biosystems). Kidney samples were evaluated for IL-21 and IL-21R whereas human cell line samples were also evaluated for FN1 and Cdh1 transcripts by qRT-PCR using Taqman primers and probes. The samples were normalized to the average of two housekeeping genes, Rpl27 and Rps13.

### Diabetic nephropathy mouse models

db/db and db/+ mice (C57BL/Ks background strain) were sacrificed at 20 weeks of age. Streptozotocin (STZ)-treated and un-treated mice (129SV and DBA/2 background strains), were sacrificed at 24 weeks of age, 15 weeks post-STZ treatment, and ob/ob and ob/+ mice (BTBR background strain), were sacrificed at 20 weeks of age. At this time all the diabetic mice displayed elevated HbA1c and albuminuria, whereas the controls all were normal.

### Flow cytometry of mouse kidney cells

Mouse kidneys were minced with a scalpel, digested with 2 mg/ml collagenase II (Gibco) and deoxyribonuclease I (Sigma, 50U/ml) for 40 min. in rolLtation at 37°C and passed through a 100 µm cell strainer. Red blood cells were lysed, and the resulting tissue suspension was again filtered (100 µm) and further digested with collagenase II (0.5 mg/ml), disIL-21Rpase II (Sigma, 0.5 mg/ml), deoxyribonuclease I (50U/ml), and trypsin (Gibco, 0.075 %) for 15 min. at 37°C. The tissue suspension was washed, resuspended in 2 mM EDTA and incubated on ice for 10 min. before being passed through a 23G needle 3 times. The resulting cell suspensions was passed through a 40 µm cell strainer and subjected to flow cytometric analysis using the following antibodies: IL-21R-PE (R&D Biosystems, clone 155516), F4/80-AlexaFluor488 (Biolegend, clone BM8), CD11b (Biolegend, M1/70), CD4-PB (Biolegend, clone RM4-5), CD45-eFluor780 (eBioscience, clone 30-F11), B220-V500 (BD Horizon, clone RA3-6B2) and unconjugated Anti-ENaCalpha (extracellular) polyclonal antibody (Alamone Labs, Jerusalem, Israel) with anti-rabbit Dyligh649 (Biolegend, clone Poly4064). Samples were analysed on a BD Fortessa using FACS Diva software (BD Biosciences). Viable cells were identified as 7-AAD-negative cells.

### Flow cytometry of cell lines

Murine podocytes (SVI immortalized cell line, Cell Lines Service, Eppelheim, Germany) and primary mouse proximal tubule epithelial cells (PTECs, ScienCell M4100) were cultured according to the vendors' instructions, stained with anti-IL-21R-APC (Biolegend, clone 4A9) and analysed by flow cytometry.

### Glomeruli apoptosis

Glomeruli were isolated from mouse kidneys using magnetic dynal beads (Life technologies, Denmark). Briefly, mice were anesthetized using isofluorane and then perfused through the left ventricle with 20 ml HBSS containing 8 x 10⁷ Dynalbeads. The kidneys were removed, digested for 25 min in 1 mg/ml collagenase A containing 100 U/ml deoxyribonuclease I at 37°C. The tissue was then passed through a 100 µm sieve and glomeruli were collected and washed using a magnetic particle concentrator. The glomeruli were then cultured on collagen I coated plates with a 10 % NuSerum F10:DMEM media and cultured for 24 h. The glomeruli were then treated with media from Th17 cells, 1:2, 1:5, and 1:10.. The glomeruli were treated for 24 h and the relative activity of caspase 3 was measured using ApoTox-Glo Triplex assay (Promega). Activated caspase 3 was used as an indicator of apoptosis.

### Induction of fibrosis and epithelial to mesechymal transition in PTEC

Human proximal tubular epithelial cells were subcultured and acclimatized for 24 h, then treated with vehicle, IL21 (200 ng/ml), IL17 (200 ng/ml) or Th17 media (5 %) for 48 h, after which the cells were lysed with RLT buffer (Qiagen, Denmark) and then stored at -20 C.

### Activation of human glomerular endothelial cells (GEC)

Human glomerular endothelial cells were subcultured and acclimatized for 24 h, then treated with IL1beta, TNFalpha, LPS and angiotensin II for 16 h. The cells were then lysed with RLT buffer (Qiagen, Denmark) and then stored at -20 C.

### Results

### Diabetic mouse kidney, primary cells and cell lines express IL-21Rα and γC mRNA

RNA from the MES13 mouse mesangial cells, SVI40 mouse podocyte cells, primary mouse proximal tubular epithelial cells (PTECs), primary human PTECs and primary human glomerular endothelial cells was analysed for the expression of IL-21 Rα and γC. The IL-21 R and IL-2y were expressed in all mouse kidney derived cell lines MES13 (IL-21Rα CT=37; γC CT=34), SVI40 (IL-21 Rα CT=32; γC CT=34) and mPTEC (IL-21 Rα CT=35; γC CT=29), and IL21 R was expressed in all human kidney cells hPTEC (IL-21 Rα CT=29) and hGEC (IL-21 Rα CT=36) (Figure 1). This demonstrated that the two receptors were consistently expressed in the kidney structural cells indicating potential responsiveness to IL-21.

Whole mouse kidney showed expression of IL-21Rα (CT=30) and γC (CT=27) (Figure 2A). Detailed analysis demonstrated that both young diabetic db/db mice glomeruli and old diabetic nephropathy db/db mice glomeruli expressed both IL-21 R and γC (Figure 2B). In the STZ induced diabetic nephropathy kidney model, IL-21 was expressed in both healthy control and diabetic mouse kidneys. In sharp contrast, IL21 R was significantly upregulated in diabetic kidneys indicating enhanced responsiveness (Figure 2C-D). These data place both receptor components in the area of the diabetic kidney associated with defective function.

### Inflammation induce IL-21R mRNA expression in human primary glomerular endothelial cells (GEC)

GEC play a key role in mediating inflammation in the glomerulus as well as maintaining the glomerular filter barrier. To determine the IL-21 R expression in GEC, the cells were stimulated with inflammatory agents like IL 1β, TNFα and LPS to mimic the local inflammatory milieu in the diabetic kidney. Upon inflammation mRNA expression of IL21R was upregulated 2-3-fold (Figure 3). These data suggests that IL21R is upregulated during an inflammatory state as is evident in the diabetic kidney.

### IL-21R is present on protein level in kidney cells

On protein level, approximately 50% of a mouse SVI podocyte cell line and 8% of human PTECs expressed IL-21 Rα as detected by surface flow cytometry (data not shown).

### Renal lymph node cells express IL-21

A higher percentage of renal lymph node (RLN) CD4+ T cells from db/db mice (10 %) stained positive for intracellular IL-21 than mesenteric lymph nodes T cells (1 %) from the same animals. Moreover, the percentage of IL-21⁺ cells was markedly higher in db/db RLN (10 % at 8 weeks of age and 6 % at 21 weeks) compared to RLN from healthy control db/+ mice (2 % at 8 weeks and 0.5 % at 21 weeks) (Figure 4A). Most importantly, the frequency of the IL-21-dependent IL-17⁺ CD4⁺ cells increased in db/db RLN (from 0 to 0.9 %), while no increase was observed in MLN (Figure 4B). In addition, no increase was observed in the control db/+ animals. Thus taken together, the local milieu in the kidney draining lymph node in diabetic nephropathy animals, but not healthy control animals, induces the IL-21-dependent Th17 subset of T cells.

### IL-21R is regulated in vivo in diabetic kidney on protein level

In kidneys from db/db and db/+ mice, the IL-21Rα protein was expressed primarily on B cells (Figure 5A) and on cells expressing the ion-channel ENaCalpha (distal tubular epithelial cells, DTECs, Figure 5B). Furthermore, IL-21Rα expression was also present in low levels on db/db and db/+ kidney T-cells (5C)

Kidney B cells and T cells in BTBR ob/ob and ob/+ mice expressed IL-21Rα. Both kidney B and T cells significantly increased their IL-21Rα expression in the diabetic BTBR ob/ob compared to healthy BTBR ob/+ control (Figure 6A, B). IL-21Rα was also expressed on kidney DTECs, but not different between db/+ and db/db mice (Figure 6C). In kidneys from STZ-treated and untreated 129SV and DBA/2 mice, IL-21Rα was primarily expressed by B cells. This IL-21Rα expression on B cells was significantly increased in kidneys from both 129SV and DBA/2 STZ diabetic mice compared to healthy control mice (Figure 7A, D). Furthermore, in diabetic DBA2 mice kidneys, treated with STZ in citrate buffer, the IL-21R expression on DTECs was increased (Figure 7E). IL-21 R was also expressed by kidney CD4⁺ T cells, but not changed in kidneys from diabetic animals. In all three diabetic nephropathy models (db/db, BTBR ob/ob and STZ) the total B cell numbers were reduced (Figure 8A-D). In the db/db and BTBR ob/ob model the total number of T cells were also reduced, whereas in the STZ model the total number of T cell was increased (Figure 9A-D). These data demonstrate that the main cell responder of IL-21, the B cell, was reduced in all diabetic models, while the main IL-21 producing cell, the T cell, was induced but only in the STZ model.

Taken together, these data show that cell types associated with disease progression in the diabetic nephropathy kidney all expressed and regulated their IL-21Rα expression.

### Glomeruli display apoptosis when exposed to Th17 conditioned medium

A hallmark of diabetic nephropathy is apoptosis of mouse glomeruli. When mouse glomeruli were exposed for media from Th17 cells for 24 h a dose-dependent induction of apoptosis was observed. In 1:2 dilution of the Th17-media a 9.1-fold increase, in the 1:5 dilution a 4.5-fold increase and in the 1:10 dilution and a 2.7-fold increase of apoptosis was observed (Figure 10). The negative control Th17 media had no effect. These data demonstrate that the IL-21 dependent Th17 subset induced a biological relevant function on a kidney cell structure and biological process well recognized in diabetic nephropathy.

### Th17 conditioned media induces inflammation and a fibrotic state in human primary proximal tubular epithelial cells

A central aspect of diabetic nephropathy is inflammation-induced interstitial fibrosis. Induction of fibrotic markers in vitro was evaluated in a human PTEC cell line after stimulation with IL-21, IL-17 or media from the IL-21 dependent Th17 T cell. Th17 media upregulated mRNA expression of fibronectin and decreased the E-cadherin expression in the cells (Figure 11). This is consistent with a differentiation of the cells into a fibrotic state suggesting that IL-21 dependent mechanisms may contribute to the fibrotic pathogenesis and EMT in diabetic nephropathy.

### Anti-IL-21 therapy for prevention of diabetic nephropathy in db/db mice

Anti-IL-21 (e.g. mAb 5 or mAb 14 type of antibodies) will be administered to STZ treated mice. The primary endpoints will be reduced albumin excretion, improved albumin creatinine ratio, reduced local inflammation, reduced expression of fibrotic and EMT markers and potentially improved histological status of the kidney. Further, supporting data will be generated by plasma and urine biomarker analysis.

### In vitro/ex vivo functional evaluation

Neutralizing anti-IL-21 will be included in the culture media when generating the Th17 media and effect on glomeruli apoptosis, PTEC fibrosis and EMT will be examined.

Whole kidney single cell suspensions from diabetic and healthy mice will be challenged with recombinant IL-21. The responsiveness to IL-21 will be determined by monitoring pSTAT expression on a cell population level by FACS.

Finally, diabetic renal fibroblasts, PTEC/DTEC and podocytes will be FACS-sorted and co-cultured with activated T cells, IL-21 and Th17 media and monitored for fibrotic and EMT markers.

### Expression of IL-21 and IL-21R positive cells in human samples

Peripheral blood and urine cells from diabetic nephropathy patients will be evaluated for expression of IL-21 and IL-21 R on protein level by FACS analysis. Furthermore, determination of functionality will be demonstrated by addition of recombinant IL-21 and pSTAT profile will be determined.

### Nephropathy:

Urinary albumin excretion rates (UAE): Increased UAE is a very early marker of diabetic kidney disease.

Transcapillary escape rate for albumin (TERalb): A measure of generalised increased leakage from the vasculature is the increased permeability for albumin. This is measured as the disappearance of I¹²⁵-labelled albumin from the blood stream. TERalb is positively correlated to UAE.

## Claims

1. An IL-21 antibody for use in treating nephropathy, wherein said antibody is an antibody that competes with a receptor for binding to IL-21.

2. An IL-21 antibody for use in treating diabetic nephropathy, wherein said antibody is an antibody that competes with a receptor for binding to IL-21.

3. An IL-21 antibody according to any one of claims 1-2, wherein the antibody competes with the IL-21 Rα for binding to IL-21.

4. An antibody according to claim 3, wherein said antibody binds to a discontinuous epitope on IL-21, wherein said epitope comprises amino acids I37 to Y52 and N92 to P108 as set forth in SEO ID NO 1.

5. An IL-21 antibody according to claim 4, wherein said antibody comprises the three CDR sequences as set forth in SEQ ID NO 2 and the three CDR sequences as set forth in SEO ID NO 3.

6. An antibody according to any one of claims 1-2, wherein said antibody is an IL-21 antibody that competes with γC for binding to IL-21.

7. An antibody according to claim 6, wherein said antibody binds to an epitope comprising amino acids Glu 65, Asp 66, Val 67, Glu 68, Thr 69, Asn 70, Glu 72, Trp 73, Lys 117, His 118, Arg 119, Leu 143, Lys 146, Met 147, His 149, Gln 150, and His 151 as set forth in SEQ ID NO.1

8. An antibody according to claim 7, wherein said antibody comprises the three CDR sequences as set forth in SEQ ID NO 4 and the three CDR sequences as set forth in SEO ID NO 5.

9. An antibody according to any one of the preceding claims, wherein said antibody is co-administered with one or more ACE inhibitors.

10. A pharmaceutical composition comprising an IL-21 antibody according to any one of claims 1-9.

11. A method of treating nephropathy, wherein said method comprises administration, to a patient in need thereof, of an appropriate amount of an IL-21 antibody, wherein said antibody competes with a receptor for binding to IL-21.

12. A method of treating diabetic nephropathy, wherein said method comprises administration, to a patient in need thereof, of an appropriate amount of an IL-21 antibody, wherein said antibody competes with a receptor for binding to IL-21.

13. A method according to any one of claim 11-12, wherein said antibody is an antibody that competes with IL-21 Rα for binding to IL-21.

14. A method according to claim 13, wherein said antibody is an antibody that binds to a discontinuous epitope on IL-21, wherein said epitope comprises amino acids I37 to Y52 and N92 to P108 as set forth in SEQ ID NO 1

15. A method according to any one of claims 11-12, wherein said antibody is an antibody that competes with γC for binding to IL-21.
